# EUROPEAN PATENT SPECIFICATION

(11) **EP 1 890 683 B1**
(45) Date of publication and mention of the grant of the patent: **08.06.2011**
(21) Application number: 06758668.5
(22) Date of filing: 27.04.2006
(51) Int. Cl.: A61K 31/095, A61P 35/00

(54) **METHODS OF SUPRESSING UV LIGHT-INDUCED SKIN CARCINOGENESIS**
VERFAHREN ZUR UNTERDRÜCKUNG VON DURCH UV-LICHT INDUZIERTER HAUTCARCINOGENESE
PROCEDES DE TRAITEMENT DE LA CARCINOGENESE DE LA PEAU INDUITE PAR LE UV

(30) Priority: 29.04.2005 US 675847 P; 15.12.2005 US 750341 P
(43) Date of publication of application: 27.02.2008
(73) Proprietor: Johns Hopkins University, Baltimore, MD 21205 (US)
(72) Inventor: TALALAY, Paul, Baltimore, MD 21210 (US); DINKOVA-KOSTOVA, Albena T., Dundee DD3 7DD (GB)
(74) Representative: Meier, Jürgen
(86) International application number: PCT/US2006/016012
(87) International publication number: WO 2006/118941

(56) References cited:
- WO-A-2004/012677
- DE-A1- 10 308 298
- MISIEWICZ ET AL: "Sulfoaphane and 2-oxohexyl isothiocyanate induce cell growth arrest and apotosis in L1210 leukemia and ME-18 melanoma cells" ONCOLOGY REPORTS, vol. 10, 2003, - 2003 pages 2045-2050, XP009070743
- TALALAY ET AL: "Mechanisms of chemoprotection by dietary constituents. sulforaphane as a case study" CANCER EPIDEMIOLOGY BIOMARKER AD PREVENTION, vol. 11, no. suppl, 2005, - 2005 pages 2790S-2792S, XP009070739

## Description

### BACKGROUND OF THE INVENTION

Skin cancer incidence is steadily rising and has reached epidemic proportions: the average rise in new skin cancer diagnoses has been 3-8% per year since the 1960s, and nonmelanoma skin cancers are now the most common types of ' cancer in the United States, with over 1 million new cases per year (1,2). This steady increase in incidence is expected to continue and is primarily due to depletion of stratospheric ozone, increased human exposure to solar radiation, and longer life expectancy. According to estimates of the National Cancer Institute, 40-50% of Americans who live to age 65 will develop skin cancer at least once and the risk of developing additional tumors is high (1,2). Thus, detailed knowledge of the potential risk factors and development of new strategies for prevention are urgently needed.

It is now widely accepted that UV radiation is the main factor responsible for the majority of nonmelanoma skin cancers. UV radiation is probably the most ubiquitous environmental carcinogen and the principal factor contributing to nonmelanoma skin cancers. At least three different effects of exposure to UV radiation contribute to the process of carcinogenesis in the skin: (i) direct DNA damage leading to the formation of DNA photoproducts, e.g., cyclobutane-pyrimidine dimers and pyrimidine-pyrimidone products (37); (ii) oxidative stress-related DNA damage resulting from formation of reactive oxygen intermediates (ROI) (39); and (iii) immunosuppression that raises tolerance to genetic instability (40). Mutations in proto-oncogenes (*ras*) as well as in tumor suppressor genes (p53 and *PTCH*) have been detected in human skin cancer samples (41,42). Point mutations in *p*53 are believed to represent an early event in many forms of carcinogenesis including the development of skin tumors (1,38,41). Cells with such mutations can give rise to clones that display genetic instability and, after clonal expansion, ultimately progress to cancers.

Prevention of skin cancer has been demonstrated in a number of animal models involving a variety of chemical carcinogens in the absence of any chemical initiators or promoters. Direct antioxidant activity, alteration of apoptosis and cell signaling pathways have been implicated in the mechanisms of inhibitory action of the preventive agents. It was shown nearly 30 years ago that some agents considered to be primarily antioxidants, e.g., butylated hydroxytoluene (BHT), significantly inhibited UV-radiation-induced erythema and tumor development in mice (5,6). The spectrum of preventive agents has gradually increased to include selenium, zinc, as well as plant antioxidants, e.g., silymarin from milk thistle, isoflavones from soybean, polyphenols from tea, and it has been proposed that their topical application could supplement the use of sunscreens in protecting the skin against UV radiation (51). Green tea, black tea, and their components, e.g., polyphenols, caffeine, and (-)-epigallocatechin gallate, effectively prevent carcinogenesis in UV light-treated high-risk mice when administered either topically or in the diet (24,25,52). Green tea polyphenol treatment also inhibits UV radiation-evoked erythema and the formation of DNA pyrimidine dimers in human skin (53). Curiously, (-)-epigallocatechin gallate, much like sulforaphane, exhibits a plethora of biological effects: antioxidant response element (ARE)-mediated induction of the phase 2 gene expression, activation of mitogen-activated protein kinases, stimulation of caspase-3 activity, and apoptosis (54). Furthermore, pretreatment of human skin with (-)-epigallocatechin gallate prevents UV-induced erythema and associated inflammation, as well as the generation of hydrogen peroxide and nitric oxide, and restores the UV-induced depletion of glutathione (GSH) and GSH peroxidase (50).

Early studies in mouse models indicated that an antioxidant-supplemented diet (e.g., one containing butylated hydroxytoluene [BHT]) significantly inhibited skin carcinogenesis that was induced either by UV radiation (4,5) or polycyclic aromatic hydrocarbons/ phorbol ester (6). BHT and other phenolic antioxidants have been shown to induce phase 2 detoxification enzymes and protect rodents against the mutagenic metabolites of benzo[a]pyrene (7). , In addition, topical or dietary administration of BHA inhibits the phorbol ester-dependent induction of ornithine decarboxylase (an early indicator of tumor promotion) in mouse epidermis (8).

The balance between intracellular processes that generate reactive intermediates (e.g., electrophiles, reactive oxygen and nitrogen species) and opposing detoxification and radical scavenging reactions determines the ultimate outcome of exposure to carcinogens (9). Devising chemical and dietary means to shift the balance towards the latter route, i.e., by induction of enzymes that catalyze phase 2 detoxification reactions, is a major strategy for protection against neoplasia (10). Especially attractive is the implementation of this approach by use of inducers that are present in edible plants because these inducers are already constituents of the human diet and are presumed to be of low toxicity.

The isothiocyanate sulforaphane is one such inducer. Sulforaphane was isolated as the principal inducer from broccoli (11) guided by the ability to induce phase 2 enzymes. The intact plant contains a precursor of sulforaphane, the glucosinolate glucoraphanin. Upon plant cell injury glucoraphanin comes in contact with the otherwise compartmentalized myrosinase, a thioglucosidase that catalyzes its hydrolysis and results in the formation of sulforaphane as a major reaction product. Subsequent studies revealed that the inducer activity in 3-day-old broccoli sprouts is 20-50 times higher than that of mature plants, and that >90% of this activity is attributable to glucoraphanin (12).

In addition to being one of the most potent naturally occurring phase 2 enzyme inducers known to date, sulforaphane exhibits additional anticancer activity. For example, sulforaphane stimulates apoptosis and inhibits proliferation (13,14), is anti-inflammatory (15) and inhibits histone deacetylase (16). In addition, sulforaphane protects several types of cultured cells against the toxicity of various biological oxidants, e.g., 4-hydroxynonenal, peroxynitrite, menadione, tent-butyl hydroperoxide (17) as well as against photo-oxidation generated by all-trans-retinaldehyde and UVA light (18).

It remains to be determined, however, whether sulforaphane can protect against UV light-induced carcinogenesis. Thus, additional testing and methods are required.

### SUMMARY

In one embodiment, administration of the isothiocyanate sulforaphane protects against UV light-induced skin carcinogenesis. In another aspect, there is provided a method of suppressing UV light-induced skin carcinogenesis in a patient comprising administering to a patient who has been exposed to UB light a therapeutically effective amount of a sulforaphane or a sulforaphane analog. In one embodiment, the sulforaphane is administered transdermally or orally. In another the sulforaphane is derived from broccoli sprouts and administered trasdermally or orally.

Sulforaphane analogs also can be employed to protect against UV light-induced skin carcinogenesis. Such sulforaphane analogs can be selected from the group consisting of 6-isothiocyanato-2-hexanone, exo-2-acetyl-6-isothiocyanatonorbornane, exo-2-isothiocyanato-6-methylsulfonylnorbornane, 6-isothiocyanato-2-hexanol, 1-isothiocyanato-4-dimethylphosphonylbutane, exo-2-(1'-hydioxyethyl)-5-isothiocyanatonorbornane, exo-2-acetyl-5-isothiocyanatonorbornane, 1-isothiocyanato-5-methylsulfonylpentane, cis-3-(methylsulfonyl)cyclohexylmethylisothiocyanate and trans-3-(methylsulfonyl)cyclohexylinethylisothiocyanate. Other isothiocyanates also can be used. Similarly, oral glucosinolates also can be employed to protect against UV light-induced skin carcinogenesis.

In another embodiment, lotions are provided for use in suppressing UV light-induced skin carcinogenesis in a patient comprising a therapeutically effective amount of suloraphane. Lotions comprising isothiocyanates or glucosinolates also are provided.

Other objects, features and advantages of the present invention will become apparent from the following detailed description. Further, the examples demonstrate the principle of the invention and cannot be expected to specifically illustrate the application of this invention to all the examples where it will be obviously useful to those skilled in the prior art.

### BRIEF DESCRIPTION OF THE DRAWINGS

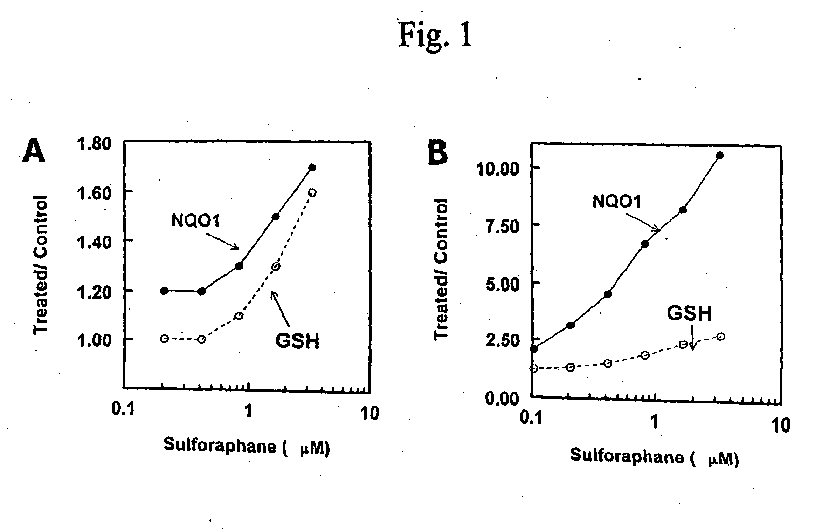

Figure 1 graphically demostrates the induction of NQO 1 (■) and elevation of GSH (O) as a function of concentration of sulforaphane in PE murine keratinocytes (A) and human HaCaT keratinocytes (B). Cells (20,000 per well) were plated on 96-well plates and exposed to a series of concentrations of sulforaphane. GSH and NQO1 levels were measured in cell lysates after 24 h and 48 h, respectively. Each data point represents the average of the measurements from 8 different wells. The standard deviation was < 5% for all data points.

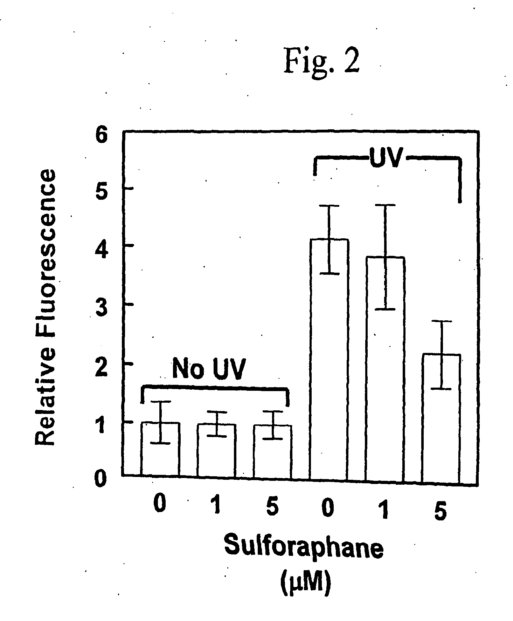

Figure 2 provides a graph showing the protection afforded by sulforaphane in PE murine keratinocytes against UVA radiation-generated reactive oxygen intermediates. Cells (50,000 per well) were plated on 24-well plates, treated with 5 µM sulforaphane for 24 h, washed with DPBS, and then exposed to UVA (10 J/cm²). Reactive oxygen intermediates generated by the UV radiation were quantified by the fluorescent probe 2',7'-dichlorodinitrofluorescein and fluorescence intensity was measured (expressed as a ratio of exposed to non-exposed cells).

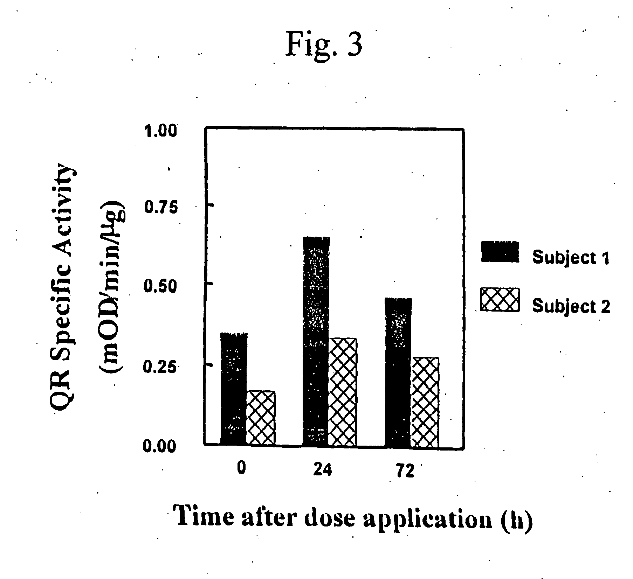

Figure 3 shows the time course of induction of quinone reductase (NQO1) in human skin of healthy human volunteers by single topical application of 100 nmol sulforaphane.

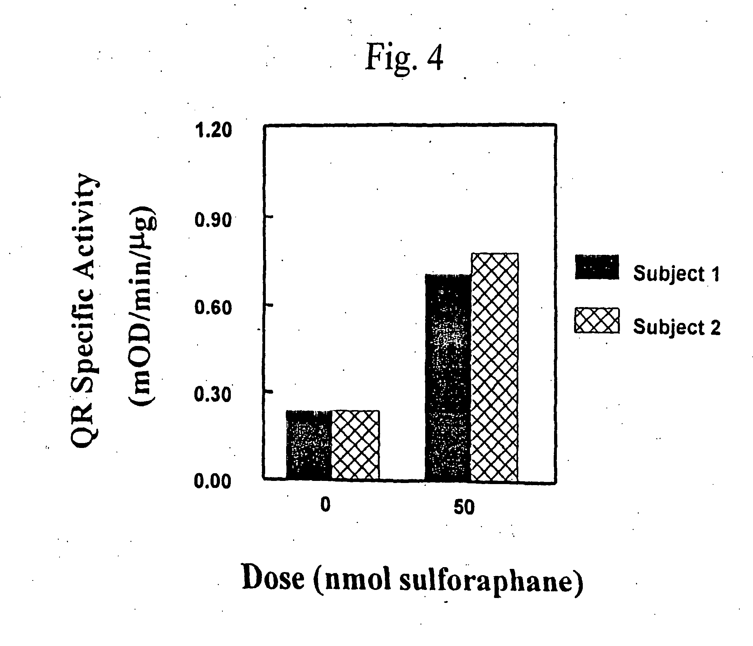

Figure 4 shows induction of NQO1 in human skin of healthy human volunteers by three repeated topical applications of 50 nmol of sulforaphane at 24 hour intervals.

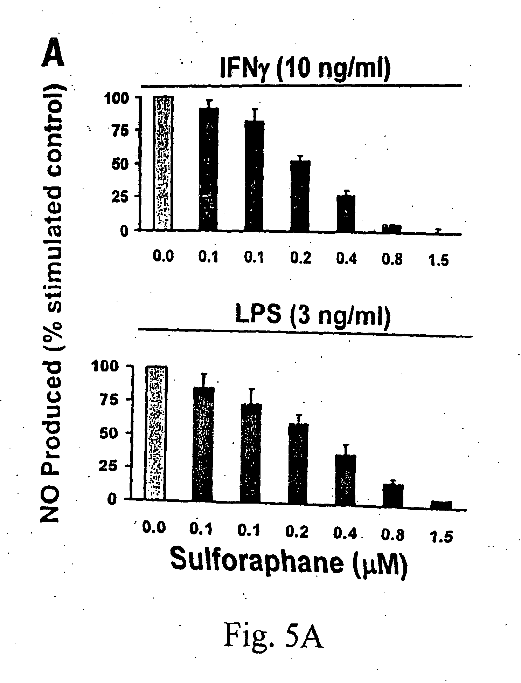

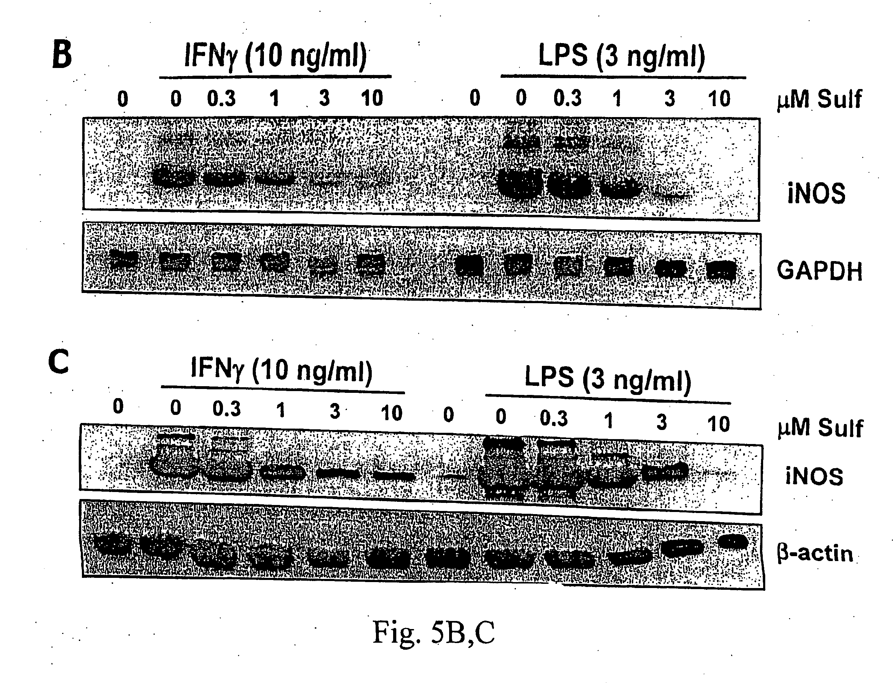

Figure 5 shows the inhibition caused by sulforaphane on (A) NO production and iNOS mRNA (B) and protein (C) induction in RAW 264.7 cells stimulated with γ-interferon or lipopolysaccharide. Cells were treated with various concentrations of sulforaphane and either IFNγ (10 ng/ml) or lipopolysaccharide (LPS; 3 ng/ml) for 24 h. NO in the medium was measured as nitrite by the Griess reaction (A), and iNOS induction was detected by Northern (B) and Western (C) blotting.

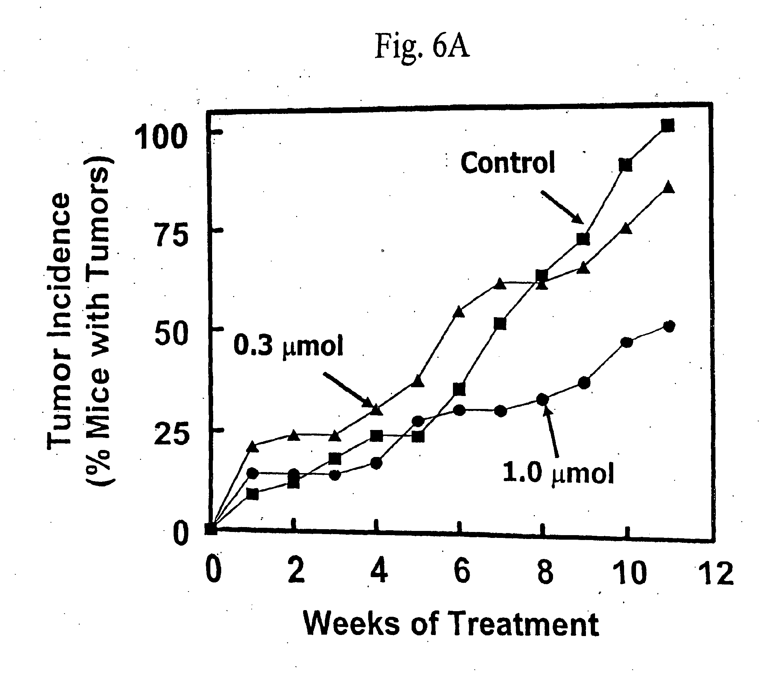

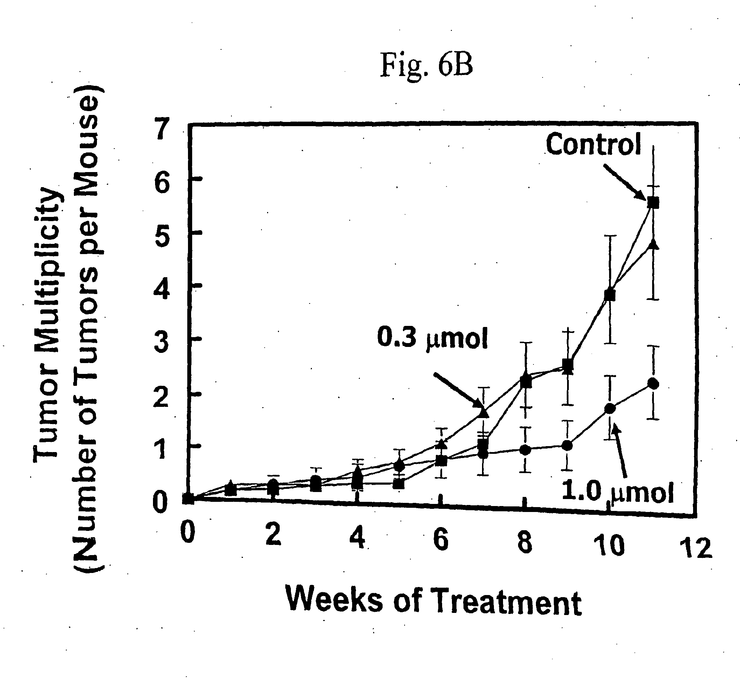

Figures 6A and 6B demonstrate the inhibition by sulforaphane of UVB radiation-induced skin carcinogenesis in high-risk mice.

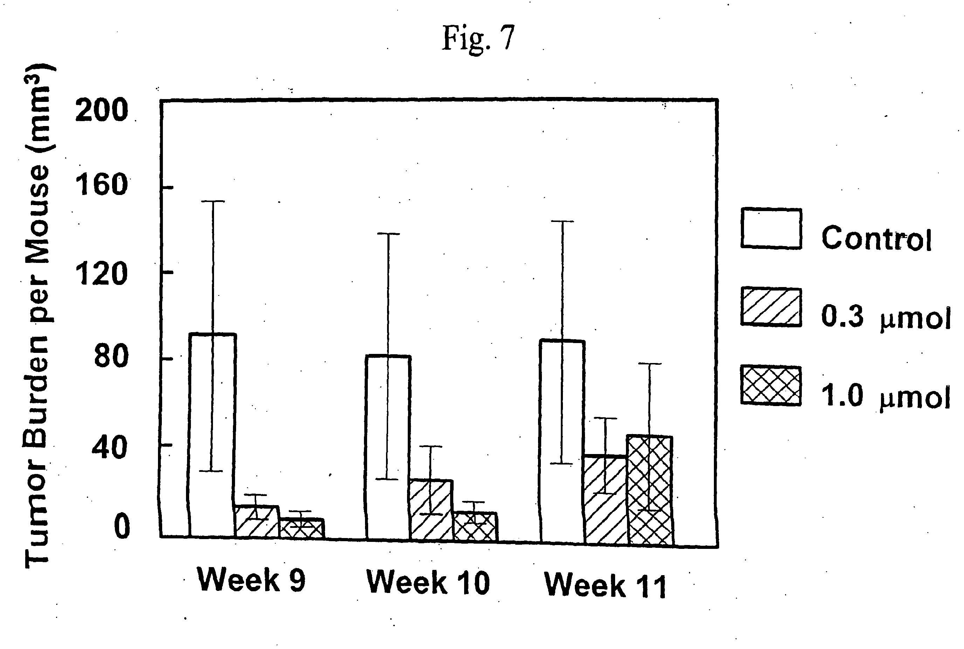

Figure 7 graphically shows the inhibition of overall tumor burden in high-risk mice by transdermal administration of sulforaphane. Tumor burden is expressed as total volume of all tumors in mm³ divided by the number of animals at risk. Average values ± SE are shown. There was a dramatic and highly significant effect (p<0.0027) of concentration (treatment) upon log transformation of tumor volume (ANOVA of concentration using treatment time as a nested variable).

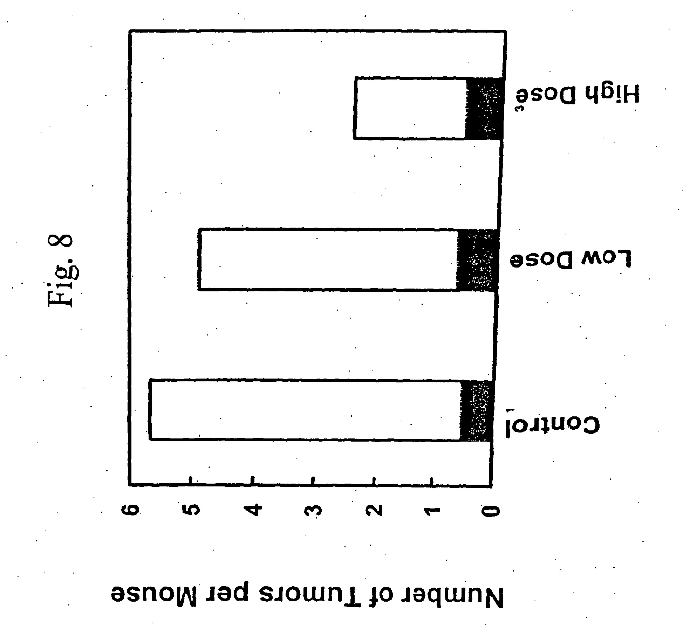

Figure 8 provides a graph showing the impact of sulforaphane on the multiplicity of small (<1 cm³, white bars) and large tumors (>1 cm³, black bars). Eleven weeks after treatment with protector or vehicle, the tumor incidence in the control group was 100%, and the experiment was terminated. All mice were euthanized on the same day and the tumor size was measured. Low dose, 0.3 µmol sulforaphane, high dose, 1.0 µmol sulforaphane applied daily, 5 times a week, to the backs of the animals.

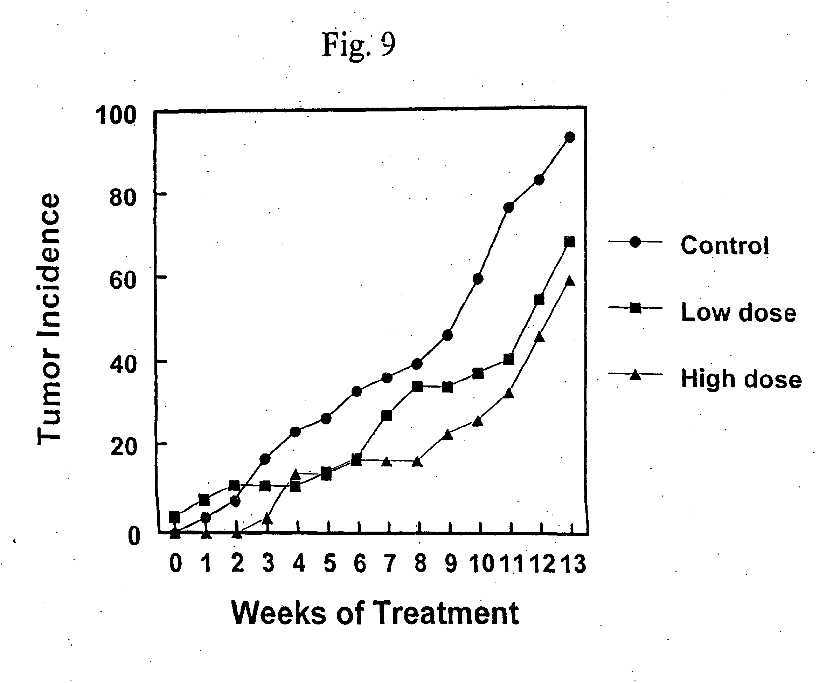

Figure 9 provides a graph showing the tumor incidence (percent mice with tumors) in high-risk mice receiving dietary administration of sulforaphane. The control group is depicted as circles, the low dose group is depicted as squares and the high dose group is depicted as triangles. Tumor incidence was reduced by 25% and 35% in the animals receiving low dose and high dose of glucoraphanin, respectively, as compared to the control group.

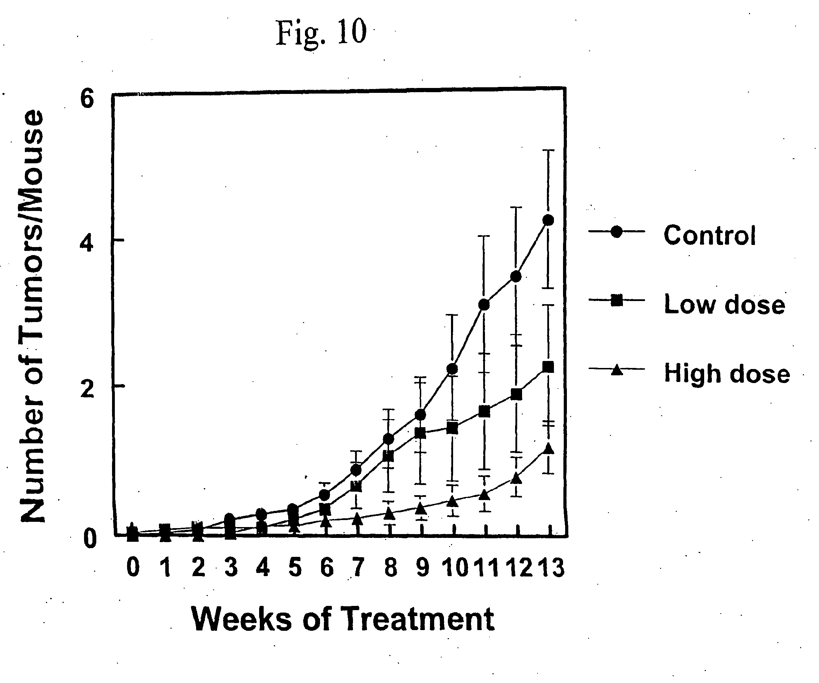

Figure 10 provides a graph showing tumor multiplicity (number of tumors per mouse) in high-risk mice receiving dietary administration of sulforaphane. The control group is depicted as circles, the low dose group is depicted as squares and the high dose group is depicted as triangles. Tumor multiplicity was reduced by 47% and 72%, respectively, as compared to the control group.

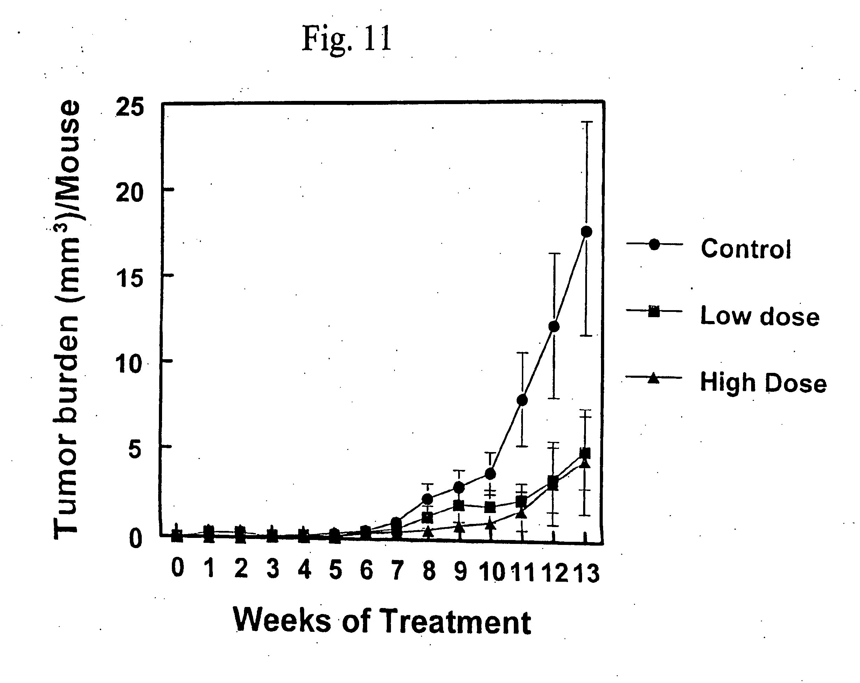

Figure 11 provides a graph showing tumor burden (total tumor volume) per mouse in high-risk mice receiving dietary administration of sulforaphane. The control group is depicted as circles, the low dose group is depicted as squares and the high dose group is depicted as triangles. Both low dose and high dose of glucoraphanin treatment resulted in 70% inhibition in the total tumor volume per mouse as compared to the control group.

### DETAILED DESCRIPTION

Administration of the isothiocyanate sulforaphane protects against UV light-induced skin carcinogenesis. In particular, topical application or dietary administration of sulforaphane after exposure to UV radiation provides effective protection against skin tumor formation.

Chemoprotective activities have been detected in certain vegetables which are able to induce the activity of enzymes that detoxify carcinogens (phase II enzymes). One such activity has been detected in broccoli which induces quinone reductase activity and glutathione S-transferase activities in murine hepatoma cells and in the organs of mice. This activity has been purified from broccoli and identified as sulforaphane. In addition, analogues of sulforaphane have been synthesized to determine structure-function relationships.

It has now been discovered that sulphoraphane provides protection against UV light-induced skin carcinogenesis. In particular, administration of sulforaphane after exposure to UV radiation provides effective protection against skin tumor formation.

Other isothiocyanates can also be employed. Isothiocyanates are compounds containing the isothiocyanate (NCS) moiety and are easily identifiable by one of ordinary skill in the art. The description and preparation of isothiocyanate analogs is described in United States Reissue Patent 36,784, and is hereby incorporated by reference in its entirety. In a preferred embodiment, the sulforaphane analogs used in the present invention include 6-isothiocyanato-2-hexanone, exo-2-acetyl-6-isothiocyanatonorbornane, exo-2-isothiocyanato-6-methylsulfonylnorbornane, 6-isothiocyanato-2-hexanol, 1-isothiocyanato-4-dimethylphosphonylbutane, exo-2-(1'-hydroxyethyl)-5-isothiocyanatonorbornane, exo-2-acetyl-5-isothiocyanatonorbornane, 1-isothiocyanato-5-methylsulfonylpentane, cis-3-(methylsulfonyl)cyclohexylmethylisothiocyanate and trans-3-(methylsulfonyl)cyclohexylmethylisothiocyanate.

In another embodiment, glucosinolates, precursors to isothiocyanates, can be used to suppress UV light-induced skin carcinogenesis. Glucosinolates are easily recognizable and appreciated by one of ordinary skill in the art and are reviewed in Fahey et al. Phytochemistry, 56:5-51 (2001), the entire contents of which are hereby incorporated by reference.

Compositions comprising sulforaphane, isothiocyanates, glucosinolates or analogs thereof can be administered in a variety of routes and comprise a variety of carriers or excipients.

By "pharmaceutically acceptable carrier" is intended, but not limited to, a non-toxic solid, semisolid or liquid filler, diluent, encapsulating material or formulation auxiliary of any type, such as liposomes.

A pharmaceutical composition of the present invention for parenteral injection can comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), carboxymethylcellulose and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

The compositions of the present invention can also contain adjuvants such as, but not limited to, preservatives, wetting agents, emulsifying agents, and dispersing agents. Prevention of the action of microorganisms can be ensured by the inclusion of various antibacterial and antifungal agents, for example, paraben, chlorobutanol, phenol, sorbic acid, and the like. It can also be desirable to include isotonic agents such as sugars, sodium chloride, and the like. Prolonged absorption of the injectable pharmaceutical form can be brought about by the inclusion of agents which delay absorption such as aluminum monostearate and gelatin.

In some cases, to prolong the effect of the drugs, it is desirable to slow the absorption from subcutaneous or intramuscular injection. This can be accomplished by the use of a liquid suspension of crystalline or amorphous material with poor water solubility. The rate of absorption of the drug then depends upon its rate of dissolution which, in turn, can depend upon crystal size and crystalline form. Alternatively, delayed absorption of a parenterally administered drug form is accomplished by dissolving or suspending the drug in an oil vehicle.

Transdermal administration of a drug is often convenient and comfortable for a patient. In this embodiment, the sulforaphane is present in a carrier. The term "carrier" refers to carrier materials suitable for facilitating transdermal drug administration, and include any such materials known in the art, e.g., any liquid, gel, solvent, liquid diluent, solubilizer, polymer or the like, which is nontoxic and which does not significantly interact with other components of the composition or the skin in a deleterious manner.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations can be sterilized, for example, by filtration through a bacterial-retaining filter, or by incorporating sterilizing agents in the form of sterile solid compositions which can be dissolved or dispersed in sterile water or other sterile injectable medium just prior to use.

Solid dosage forms for oral administration include, but are not limited to, capsules, tablets, pills, powders, and granules. In such solid dosage forms, the active compounds are mixed with at least one item pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, acetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form can also comprise buffering agents.

Solid compositions of a similar type can also be employed as fillers in soft and hard filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They can optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

The active compounds can also be in micro-encapsulated form, if appropriate, with one or more of the above-mentioned excipients.

Liquid dosage forms for oral administration include, but are not limited to, pharmaceutically acceptable emulsions, solutions, suspensions, syrups and elixirs. In addition to the active compounds, the liquid dosage forms can contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylcne glycol, dimethyl formamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, and perfuming agents.

Suspensions, in addition to the active compounds, can contain suspending agents as, for example, ethoxylated isostearyl alcohols, polyoxyethylene sorbitol and sorbitan esters, microcrystalline cellulose, aluminum metahydroxide, bentonite, agar-agar, and tragacanth, and mixtures thereof.

A dietary composition according to the present invention is any ingestible preparation containing sulforaphane, isothiocyanates, glucosinolates or analogs thereof. For example, sulforaphane, isothiocyanates, glucosinolates or analogs thereof may be mixed with a food product. The food product can be dried, cooked, boiled, lyophilized or baked. Breads, teas, soups, cereals, salads, sandwiches, sprouts, vegetables, animal feed, pills, and tablets, are among the vast number of different food products contemplated.

One of ordinary skill in the art will appreciate that effective amounts of the agents of the invention can be determined empirically and can be employed in pure form or, where such forms exist, in pharmaceutically acceptable salt, ester or prodrug form. A "therapeutically effective" amount of the inventive compositions can be determined by prevention or amelioration of adverse conditions or symptoms of diseases, injuries or disorders being treated. The agents can be administered to a subject exposed to UV radiation as pharmaceutical compositions in combination with one or more pharmaceutically acceptable excipients. It will be understood that, when administered to a human patient, the total daily usage of the agents or composition of the present invention will be decided by the attending physician within the scope of sound medical judgement. The specific therapeutically effective dose level for any particular patient will depend upon a variety of factors: the type and degree of the cellular or physiological response to be achieved; activity of the specific agent or composition employed; the specific agents or composition employed; the age, body weight, general health, sex and diet of the patient; the time of administration, route of administration, and rate of excretion of the agent; the duration of the treatment; drugs used in combination or coincidental with the specific agent; and like factors well known in the medical arts. For example, it is well within the skill of the art to start doses of the agents at levels lower than those required to achieve the desired therapeutic effect and to gradually increase the dosages until the desired effect is achieved.

The potential commercial uses of the disclosed preparations include, for example, (i) protective/prophylactic, (ii) cosmetic and (iii) medical. In one embodiment, protective lotions and crèmes for topical application either oil-(sulforaphane) or water-based (glucoraphanin plus hydrolyzing agent) are provided. In another embodiment, sulforaphane-containing compositions can be combined with sunscreens.

### Examples

### Example 1. Preparation of sulforaphane from broccoli sprouts

Seeds of broccoli (*Brassica oleracea italica,* cv. DeCicco), certified not to have been treated with any pesticides or other seed treatment chemicals, were sprouted and processed as described by Fahey et al. (12). Briefly, seeds were surface-disinfected with a 25% aqueous solution of Clorox® bleach containing a trace of Alconox® detergent and exhaustively rinsed with water. The seeds were then spread out in a layer in inclined, perforated plastic trays, misted with filtered water for 30 s about 6 times/h and illuminated from overhead fluorescent lamps. Growth was stopped after 3 days by plunging sprouts directly into boiling water in a steam-jacketed kettle, returning to a boil, and stirring for ∼5 min. This treatment inactivated the endogenous sprout myrosinase and extracted the glucosinolates. Glucoraphanin, the precursor of sulforaphane, was the predominant glucosinolate in the initial extract as determined by HPLC (26). Daikon sprout myrosinase was then added for quantitative conversion of glucosinolates to isothiocyanates as described by Fahey et al., 1997 and Shapiro et al.; 2001 (12,27). This preparation was then lyophilized, dissolved in ethyl acetate, evaporated to dryness by rotary evaporation, dissolved in a small volume of water, and acetone was added to a final concentration of 50 mM sulforaphane in 80% acetone:20% water (v/v). The total isothiocyanate content was determined (12,27) by the cyclocondensation reaction (28), complete absence of glucosinolates was confirmed by HPLC (26), and the precise ratio of the isothiocyanates liberated by the myrosinase reaction was determined by HPLC on an acetonitrile gradient, and matched the glucosinolate profile of the extract. Sulforaphane constituted more than 90 % of the isothiocyanate content. This preparation was diluted in 80 % acetone (v/v) to produce the "high dose" (1.0 µmol/ 100 µl) and "low dose" (0.3 µmol/ 100 µl). Bioassay in the Prochaska test (29,30) yielded a CD value (concentration required to double the activity of NQO1) consistent with previous experiments (11).

### Example 2 Treatment of keratinocytes with sulforaphane

Glutathione is the primary and most abundant cellular nonprotein thiol and constitutes a critical part of the cellular defense: it reacts readily with potentially damaging electrophiles and participates in the detoxification of reactive oxygen intermediates and their toxic metabolites by scavenging free radicals and reducing peroxides. The capacity to increase cellular levels of GSH is critically important in combating oxidative stress. To this end, we examined the ability of the sulforaphane-induced phase 2 response to protect against oxidative stress caused by UVA in cultures of keratinocytes. We chose UVA for this study, because its genotoxicity is thought to be primarily due to the generation of reactive oxygen intermediates.

### Cell cultures

HaCaT human keratinocytes (a gift from G. Tim Bowden, Arizona Cancer Center, Tucson) were cultured in Dulbecco's modified Eagle's medium (DMEM) supplemented with 5% FBS; and PE murine keratinocytes (a gift from Stuart H. Yuspa, National Cancer Institute, Bethesda, MD) were cultured in Eagle's minimum essential medium (EMEM) with 8% FBS, treated with Chelex resin (Bio-Rad) to remove Ca²⁺_{.}

### Quinone reductase (NQO1) and glutathione assays

Cells (20,000 per well) were grown for 24 h in 96-well plates, then exposed to serial dilutions of sulforaphane for either 24 h (for glutathione determination) or 48 h (for NQO1 determination), and finally lysed in 0.08% digitonin. An aliquot (25 µl) was used for protein analysis. Activity of NQO1 was determined by the Prochaska test (29,30). To measure the intracellular glutathione levels, 25 µl of cell lysate received 50 µl of ice-cold metaphosphoric acid (50 g/liter) in 2 mM EDTA to precipitate cellular protein. After 10 min at 4°C, plates were centrifuged at 1,500 g for 15 min and 50 µl of the resulting supernatant fractions were transferred to a parallel plate. To each of these wells, 50 µl of 200 mM sodium phosphate buffer, pH 7.5, containing 10 mM EDTA, were added and total cellular glutathione was determined by rate measurements in a recycling assay (31,32).

### UV irradiation of cells and determination of reactive oxygen intermediates

PE cells (50,000 per well) were seeded into 24-well plates and grown for 48 h. The cells were then exposed to 1 µM or 5 µM sulforaphane for 24 h. On the day of the experiments, after removing the medium, the cells were incubated with 100 µM 2',7'-dichlorodinitrofluorescein diacetate in 500 µl of fresh medium (Molecular Probes, Eugene, OR) for 30 min. The medium containing the fluorescent probe was then removed, the cells were washed with DPBS, and exposed to UVA radiation (10 J/cm²). Control cells were kept in the dark. Cells were detached with trypsin, suspended in 2.0 ml of DPBS, and the intensity of fluorescence was determined in cell suspensions at 520 nm with an excitation of 485 nm in 2-ml cuvettes in a Perkin-Elmer LS50 spectrofluorimeter.

When HaCaT human keratinocytes or PE murine keratinocytes were exposed to sulforaphane, the intracellular levels of NQO1 and glutathione were increased in a dose-dependent manner (Fig. 1A, B) in agreement with previous observations (Ye and Zhang, 2001). Especially striking was the magnitude of NQO1 induction (>10-fold) in HaCaT cells without any apparent evidence of cytotoxicity. Treatment with 5 µM sulforaphane for 24 h produced a substantial (50%) reduction in reactive oxygen intermediates generated by the UV radiation as quantified by the fluorescent probe 2',7'-dichlorodinitro-fluorescein (35) (Fig. 2).

### Example 3. Effect of topical application of sulforaphane on NQO1 and GSH in mice

The phase 2 response was next evaluated *in vivo* in SKH-1 hairless mice. Female SKH-1 hairless mice (4 weeks old) were obtained from Charles River Breeding Laboratories (Wilmington, MA) and were acclimatized in our animal facility for 2 weeks before the start of the experiment. The animals were kept on a 12-h light/12-h dark cycle, 35% humidity, and given free access to water and pelleted AIN 76A diet (Harlan TekLad, free of inducers). All animal experiments were in compliance with the National Institutes of Health Guidelines and were approved by the Johns Hopkins University Animal Care and Use Committee.

Seven-week-old SKH-1 hairless mice (5 per group) were treated topically on their backs with either 100 µl of a standardized myrosinase-hydrolyzed broccoli sprout extract containing 1 µmol of sulforaphane, or vehicle (100 µl of 80% acetone : 20% water, v/v). The animals were euthanized 24 h later and their dorsal skins were dissected using a rectangular template (2.5 x 5 cm) and frozen in liquid N₂. Skin samples were pulverized in liquid N₂ and 100 mg of the resulting powder was homogenized in 1 ml of either 0.25 M sucrose buffered with 10 mM Tris-HCl, pH 7.4, for analysis of NQO1 enzymatic activity and protein content, or ice-cold metaphosphoric acid (50 g/liter) in 2 mM EDTA for analysis of glutathione. Centrifugation at 14,000 g for 20 min at 4 °C yielded clear supernatant fractions, aliquots of which were used for determination of protein content, enzyme activity, and total glutathione levels as described below for the cell culture experiments.

The results showed that topical administration of sulfopharane produced about a 50 % induction of NQO1 (P< 0.001) and about a 15 % elevation of the total glutathione levels of the treated animals compared to the controls.

### Example 4. Effect of topical application of sulforaphane on NQO1 and GSH in humans

This study involving healthy human volunteers was done in accordance with protocols approved by the Institutional Review Board at the Johns Hopkins University. The safety of topical administration of single doses of broccoli sprout extracts to the skin of healthy human volunteers was studied. The extracts were prepared in 80% acetone : 20% water and their sulforaphane content was precisely determined by cyclocondensation assay, a method routinely used in our laboratory for quantification of isothiocyanates and their dithiocarbamate metabolites. A circle (1 cm in diameter) was drawn on the skin of volar forearm of each participant and the extract was then applied inside the circle by using a positive displacement pipette. Two subjects participated for each of the 8 escalating doses that were administered (0.3; 5.3; 10.7; 21.4; 42.7; 85.4; 170; and 340 nmol of sulforaphane). Each subject served as his/her own control and received a placebo "vehicle spot." No adverse reactions were observed at any of these doses.

Efficacy studies were also performed. The endpoint was determination of the enzyme activity of quinone reductase (a prototypic Phase 2 protein) in 3-mm skin punch biopsies of 2 healthy human volunteers after application of a single dose of broccoli sprout extract. Again, each subject served as his/her own control and received a "vehicle spot". Both quinone reductase activity and protein content were reliably detected in these samples. The specific activity of quinone reductase was increased by ∼2-food 24 h after application of an extract containing 100 nmol of sulforaphane (Fig. 3). Notably, the induction was long-lasting as the activity remained higher than that of the placebo-treated sites even when the biopsies were performed 72 h after application.

The effect of three repeated topical applications (at 24-h intervals) of broccoli sprout extract containing 50 nmol of sulforaphane was studied next. This led to even greater elevations of quinone reductase (NQO1) specific activity in the underlying skin of two healthy human volunteers (Fig. 4).

### Example 5. Effect of sulforaphane on inducible nitric oxide synthase

We have recently found a linear correlation spanning over 6 orders of magnitude of potencies between inhibition of inflammatory responses (iNOS and COX-2 activation by γ-interferon) and induction of phase 2 enzymes among a series of synthetic triterpenoids (20).

RAW 264.7 macrophages (5 x 10⁵ cells/well) were plated in 96-well plates and incubated with sulforaphane and either 10 ng/ml of IFN-γ or 3 ng/ml of LPS for 24 h. NO was measured as nitrite by the Griess reaction (33). When RAW 264.7 cells were incubated with γ-interferon or lipopolysaccharide together with various concentrations of sulforaphane for 24 h, there was a dose-dependent inhibition of NO formation with an IC₅₀ of 0.3 µM for both cytokines (Fig. 5A).

In agreement with this result, Northern and Western blot analyses revealed that the synthesis of iNOS mRNA and protein were also inhibited (Fig. 5B,C). RAW 264.7 macrophages (2 x 10⁶ cells/well) were incubated with sulforaphane and either 10 ng/ml of IFN-γ or 3 ng/ml of LPS overnight. For Northern blots, total RNA was isolated with Trizol reagent (Invitrogen) and prepared for blotting as previously described (33). Probes for iNOS and GAPDH were radiolabeled with [y-³²P]dCTP with random primers. For Western blots, total cell lysates were subjected to SDS/PAGE, transferred to a membrane, and probed with iNOS and β-actin antibodies (Santa Cruz Biotechnology).

These findings indicate that exposure to sulforaphane suppresses induction of iNOS by either γ-interferon or lipopolysaccharide and attenuates inflammatory responses that play a role in the process of carcinogenesis.

### Example 6. Effect of topical applications of sulforaphane on UV light-inducted carcinogenesis

Exposure of SKH-1 hairless mice to relatively low doses of UVB radiation (30 mJ/ cm²) twice a week for 20 weeks results in "high-risk mice" that subsequently develop skin tumors in the absence of further UV treatment (24,25). This animal model is highly relevant to humans who have been heavily exposed to sunlight as children, but have limited their exposure as adults. In addition, it allows the evaluation of potential chemoprotective agents after completion of the irradiation schedule, thus excluding the possibility of a "light filtering effect" by the protective preparations of sprout extracts that may be slightly colored. Thus, UVB-retreated high-risk mice were treated topically once a day 5 days a week for 11 weeks with 100 µl of standardized myrosinase-hydrolyzed broccoli sprout extracts containing either 0.3 µmol (low dose) or 1 µmol (high dose) of sulforaphane. The control group received vehicle treatment. Body weights and formation of tumors larger than 1 mm in diameter were determined weekly.

UVB radiation was provided by a bank of UV lamps (FS72T12-UVB-HO, National Biological Corporation, Twinsburg, OH) emitting UVB (280-320 nm, 65% of total energy) and UVA (320-375 nm, 35% of total energy). The radiant dose of UVB was quantified with a UVB Daavlin Flex Control Integrating Dosimeter and further calibrated with an IL-1400 radiometer (International Light, Newburyport, MA).

The animals were irradiated for 20 weeks on Tuesdays and Fridays with a radiant exposure of 30 mJ/cm²/session. One week later, the mice were divided into three groups: 29 animals in each treatment group and 33 animals in the control group. The mice in the two treatment groups received topical applications of either 100 µl of broccoli sprout extract containing 1 µmol sulforaphane (high dose), or 0.3 µmol of sulforaphane (low dose), those in the control group received 100 µl of vehicle. Treatment was repeated 5 days a week for 11 weeks at which time all animals in the control group had at least one tumor and the experiment was ended. Tumors (defined as lesions > 1 mm in diameter) and body weight were recorded weekly. Tumor volumes were determined by measuring the height, length, and width of each mass that was larger than 1 mm in diameter. The average of the three measurements was used as the diameter and the volume was calculated (v = 4πr³/3). All mice were euthanized on the same day and the size and multiplicity of tumors was determined. Dorsal skins were dissected using a rectangular template (2.5 x 5 cm) to include the entire treated areas of the mice. Skins were stapled to cardboard, photographed, and fixed in ice-cold 10% phosphate-buffered formalin at 4 °C for 24 h.

There was no difference in average body weight and weight gain among the groups. The body weights (mean ± SD) at the onset of the experiment were: 22.3 ± 1.9 g for the control group, 22.2 ± 1.9 g for the low-dose-treated, and 23.0 ± 1.9 g for the high dose-treated group. At the end of the experiment (31 weeks later), the respective body weights were: 32.1 ± 9.7 g, 31.9 ± 8.8 g, and 32.1 ± 6.9 g. The earliest lesions larger than 1 mm were observed 2 weeks after the end of irradiation which was 1 week after topical treatment with protector was started. At this time point, 3, 6, and 4 mice of the control, low dose-treated, and high dose-treated mice, respectively, developed their first tumor.

The high dose-treated animals were substantially protected against the carcinogenic effects of UV radiation. Thus, after 11 weeks of treatment when the experiment was terminated, 100% of the animals in the control group had developed tumors, while 48% of the mice treated daily with sprout extract containing 1 µmol of sulforaphane were tumor-free (Fig. 6A). Of note, three animals (two of the control and one of the low-dose-treated groups) were euthanized 1 week before the end of the experiment because they had tumors approaching 2 cm in diameter. Kaplan-Meier survival analysis followed by both a stratified log-rank test, and a Wilcoxon test for equality of survivor functions showed that there was a highly significant difference (P<0.0001) between treatments. The 1-µmol treatment was different from both the 0.3 µmol and the control treatment, at the 95% confidence level, for each of the last three observation periods (weeks 9, 10, and 11). There was no significant difference between the 0.3 µmol and the control treatment at any time point.

Figure 6B shows the overall effect of treatment on tumor number was highly significant (p<0.001). ANOVA comparisons of the 1.0-µmol dose level with the control indicated a highly significant overall effect (p<0.001), but differences only became significant after week 9: p<0.0794, p<0.0464 and p<0.0087 for observations made at weeks 9, 10, and 11, respectively. Average values ± SE are shown.

In addition to the reduction in tumor incidence and multiplicity, there was a significant delay of tumor appearance. Whereas 50% of the control animals at risk had tumors at 6.5 weeks after the end of radiation, it took 10.5 weeks for 50% of the high-dose treated animals at risk to develop tumors. Of note, the ability of a protective agent to delay the carcinogenic process is becoming an increasingly appreciated concept in chemoprevention. Similarly, tumor multiplicity was reduced by 58%: the average number of tumors per mouse was 2.4 for the treated and 5.7 for the control group.

Although there was no difference in tumor incidence and multiplicity between the low-dose-treated and the vehicle-treated groups (Fig. 6A, B), the overall tumor burden (expressed as volume in mm³) per mouse was substantially smaller in the low dose-treated group by 86-, 68-, and 56% at treatment weeks 9, 10, and 11, respectively (Fig. 7). The seemingly decreasing effectiveness with respect to treatment with time appears to occur because the large tumors (>1 cm³) grew rapidly during the last 2 weeks of the experiment. The overall tumor burden in the high dose-treated group was even more dramatically reduced by 91-, 85-, and 46% at treatment weeks 9, 10, and 11, respectively. Interestingly, some of the mice from this treatment group had tumors on the head, where the extract was not applied, but no tumors on their back, where the protective extract was applied.

Although histological characterization of the individual tumors has not been completed, this animal model consistently results in the formation of approximately 80% small nonmalignant tumors (primarily keratoacanthomas and a few papillomas) and approximately 20% large malignant tumors (squamous cell carcinoma) (24,25). We classified all tumors according to their volumes in two categories: "small" (<1 cm³) (Fig. 8, white bars) and "large" (>1 cm³) (Fig. 8, black bars). Treatment with the sprout extract did not change the multiplicity of large tumors across the experimerital groups, there were 17 large tumors among all 33 animals in the control group, 19 among all 29 animals in the low dose-treated group, and 16 among all 29 animals in the high dose-treated group. In contrast, the broccoli sprout extract produced a dose-dependent inhibition on the number of small tumors: 170, 123, and 54 in the control, low dose-treated, and high dose-treated groups, respectively. It is possible that the unaffected tumors originated from cells that had accumulated mutations caused by direct UV-radiation-induced DNA photoproducts, whereas the extracts inhibited mainly carcinogenic processes resulting from oxidative stress-induced DNA damage. A similar phenomenon has been reported in that the soybean isoflavone genistein inhibited the generation of lipid peroxidation products, H₂O₂, and 8-hydroxy-2'-deoxyguanosine in mouse skin, but had no effect on the pyrimidine dimers formed in response to UV radiation (36).

### Statistical analysis

Tumor incidence was evaluated using the Kaplan-Meier survival analysis followed by both a stratified log-rank test and a Wilcoxon test, for equality of survivor functions. Tumor multiplicity was evaluated by ANOVA and comparisons were made on all treatments and on individual, paired treatments (t-test). Tumor volume was evaluated by ANOVA with treatment time as a nested variable. These calculations were performed using Stata 7.0 (College Station, TX). Other statistics were calculated using Excel.

### Example 7: Preparation of freeze-dried broccoli sprout extract powder

Seeds of broccoli (*Brassica oleracea italica,* cv. DeCicco) were used to grow sprouts as described in Example 1. Growth was arrested after 3 days by plunging sprouts into boiling water and allowed to boil for ∼30 min. This treatment inactivated the endogenous sprout myrosinase and extracted the glucosinolates. Glucoraphanin, the precursor of sulforaphane, was the predominant glucosinolate in the extract as determined by HPLC (26). This preparation was then lyophilized to give glucosinolate-rich powder that contained ∼8.8 % of glucoraphanin by weight. The powder was mixed with the mouse diet (powdered AIN 76A) to give the equivalent of 10 µmol (low dose) or 50 µmol (high dose) of glucoraphanin per 3 grams of diet.

### Example 8: Effect of dietary administration of sulforaphane on UV light-induced carcinogenesis

In this study, UVB-pretreated high-risk mice were fed for 13 weeks a diet into which was incorporated a freeze-dried broccoli sprout extract powder prepared according to Example 6 (equivalent to 10 µmol/day [low dose] and 50 µmol/day [high dose] glucoraphanin, the glucosinolate precursor of sulforaphane that is found in the intact plant, about 10% of which is converted to sulforaphane upon ingestion by mice). The diet of the control group did not contain any freeze-dried broccoli sprout extract powder. Body weights and formation of tumors larger than 1 mm in diameter were determined weekly.

UVB radiation was provided by a bank of UV lamps (FS72T12-UVB-HO, National Biological Corporation, Twinsburg, OH) emitting UVB (280-320 nm, 65% of total energy) and UVA (320-375 nm, 35% of total energy). The radiant dose of UVB was quantified with a UVB Daavlin Flex Control Integrating Dosimeter and further calibrated with an IL-1400 radiometer (International Light, Newburyport, MA).

The animals were irradiated for 20 weeks on Tuesdays and Fridays with a radiant exposure of 30 mJ/cm²/session. One week later, the mice were divided into three groups: 30 animals in each treatment group and 30 animals in the control group. The mice in the two treatment groups received a diet into which was incorporated a freeze-dried broccoli sprout extract powder. The diet of the low dose treatment group included a freeze-dried broccoli sprout extract powder equivalent to 10 µmol/day glucoraphanin, while the diet of the high dose treatment group included a freeze-dried broccoli sprout extract powder equivalent to 50 µmol/day glucoraphanin. The diet of the control group did not contain a freeze-dried broccoli sprout extract powder. The mice were fed this diet for 13 weeks. After 13 weeks, 93% of the control mice had tumors and the experiment was ended.

Tumor volumes were determined by measuring the height, length, and width of each mass that was larger than 1 mm in diameter. The average of the three measurements was used as the diameter and the volume was calculated (v = 4πr³/3). All mice were euthanized on the same day and the size and multiplicity of tumors was determined. Dorsal skins were dissected using a rectangular template (2.5 x 5 cm) to include the entire treated areas of the mice. Skins were stapled to cardboard, photographed, and fixed in ice-cold 10% phosphate-buffered formalin at 4°C for 24 h.

Tumor incidence (percent animals with tumors) was reduced by 25% and 35%, in the animals receiving the low dose and the high dose of glucoraphanin, respectively, as compared to the control group of mice. (Fig. 9)

Even greater was the effect of treatment on tumor multiplicity (number of tumors per mouse) that was reduced by 47% and 72% in the animals receiving the low dose and the high dose of glucoraphanin, respectively, as compared to the control group of mice. Thus, while the animals in the control group had on the average of 4.3 tumors per mouse, the number of tumors per mouse was 2.3 for the low dose and 1.2 for the high dose of glucoraphanin. (Fig. 10)

Tumor burden was also affected dramatically: both low dose and high dose of glucoraphanin treatments resulted in 70% inhibition in the total tumor volume per mouse. (Fig. 11)

The plasma levels of sulforaphane and its metabolites were very similar: 2.2 µM and 2.5 µM for the low dose and the high dose of glucoraphanin treatments, respectively, indicating that glucoraphanin was converted to sulforaphane and that the chronic dietary treatment had resulted in steady-state levels of sulforaphane and its metabolites in the blood of the animals. These levels are adequate to expect biological effects.

The levels of phase 2 enzymes were induced (2 to 2.5-fold for quinone reductase 1 and 1.2 to 2.2-fold for glutathione S-transferases) in nearly all the organs that were examined, namely forestomach, stomach, bladder, liver, and retina.

### Statistical analysis

Tumor incidence was evaluated using the Kaplan-Meier survival analysis followed by both a stratified log-rank test and a Wilcoxon test, for equality of survivor functions. Tumor multiplicity was evaluated by ANOVA and comparisons were made on all treatments and on individual, paired treatments (t-test). Tumor volume was evaluated by ANOVA with treatment time as a nested variable. These calculations were performed using Stata 7.0 (College Station, TX). Other statistics were calculated using Excel.

In conclusion, topical or dietary administration of broccoli sprout extracts as a source of sulforaphane in the diet protects against skin tumor formation in a mouse model that is highly relevant to human exposure to UV light.

This invention was made with government support under CA06973 and CA93780 awarded by the National Cancer Institute. The government has certain rights in the invention.

Abbreviations: COX-2, cyclooxygenase 2; GSH, glutathione; γ-IFN, interferon γ; iNOS, inducible nitric oxide synthase; LPS, lipopolysaccharide; NQO1, NAD(P)H-quinone acceptor oxidoreductase, also designated quinone reductase.

### REFERENCES

1. Alam,M. and Ratner,D. (2001) Cutaneous squamous-cell carcinoma. N. Engl. J. Med., 344, 975-983.
2. Diepgen,T.L. and Mahler, V. (2002) The epidemiology of skin cancer. Br. J. Dermatol., 146, 1-6.
3. Peak,M.J., Peak,J.G. and Carnes, B.A. (1987) Induction of direct and indirect single-strand breaks in human cell DNA by far- and near-ultraviolet radiations: action spectrum and mechanisms. Photochem. Photobiol., 45, 381-387.
4. Black,H.S. and Chan,J.T. (1975) Suppression of ultraviolet light-induced tumor formation by dietary antioxidants. J. Invest. Dermatol., 65, 412-414.
5. Black,H.S., Chan,J.T. and Brown,G.E. (1978) Effects of dietary constituents on ultraviolet light-mediated carcinogenesis. Cancer Res., 38, 1384-1387.
6. Slaga,T.J. and Bracken,W.M. (1977) The effects of antioxidants on skin tumor initiation and aryl hydrocarbon hydroxylase. Cancer Res., 37, 1631-1635.
7. Talalay,P., Batzinger,R.P., Benson,A.M., Bueding,E. and Cha,Y.N. (1979) Biochemical studies on the mechanisms by which dietary antioxidants suppress mutagenic activity. Adv. Enzyme. Regul., 17, 23-36.
8. Kozumbo,W.J., Seed,J.L. and Kensler,T.W. (1983) Inhibition by 2(3)-tert-butyl-4-hydroxyanisole and other antioxidants of epidermal ornithine decarboxylase activity induced by 12-O-tetradecanoylphorbol-13-acetate. Cancer Res., 43, 2555-2559.
9. Talalay,P. (1989) Mechanisms of induction of enzymes that protect against chemical carcinogenesis. Adv. Enzyme Regul., 28, 237-250.
10. Talalay,P. (2000) Chemoprotection against cancer by induction of phase 2 enzymes. Biofactors, 12, 5-11.
11. Zhang,Y., Talalay, P., Cho,C.G. and Posner,G.H. (1992) A major inducer of anticarcinogenic protective enzymes from broccoli: isolation and elucidation of structure. Proc. Natl. Acad. Sci. USA, 89, 2399-2403.
12. Fahey,J.W., Zhang,Y. and Talalay,P. (1997) Broccoli sprouts: an exceptionally rich source of inducers of enzymes that protect against chemical carcinogens. Proc. Natl., Acad Sci. USA, 94, 10367-10372.
13. Gamet-Payrastre,L., Li,P., Lumeau,S., Cassar,G., Dupont,M.A., Chevolleau,S., Gasc,N., Tulliez,J. and Tercé,F. (2000) Sulforaphane, a naturally occurring isothiocyanate, induces cell cycle arrest and apoptosis in HT29 human colon cancer cells. Cancer Res., 60, 1426-1433.
14. Choi,S. and Singh,S.V. (2005) Bax and Bak are required for apoptosis induction by sulforaphane, a cruciferous vegetable-derived cancer chemopreventive agent. Cancer Res., 65, 2035-2043.
15. Heiss,E., Herhaus,C., Klimo,K., Bartsch,H. and Gerhauser,C. (2001) Nuclear factor kappa B is a molecular target for sulforaphane-mediated anti-inflammatory mechanisms. J. Biol. Chem., 276, 32008-32015.
16. Myzak,M.C., Karplus,P.A., Chung,F.L. and Dashwood,R.H. (2004) A novel mechanism of chemoprotection by sulforaphane: inhibition of histone deacetylase. Cancer Res., 64, 5767-5774.
17. Gao,X., Dinkova-Kostova,A.T. and Talalay,P. (2001) Powerful and prolonged protection of human retinal pigment epithelial cells, keratinocytes, and mouse leukemia cells against oxidative damage: the indirect antioxidant effects of sulforaphane. Proc. Natl. Acad. Sci. USA, 98,15221-15226.
18. Gao,X. and Talalay,P. (2004) Induction of phase 2 genes by sulforaphane protects retinal pigment epithelial cells against photooxidative damage. Proc. Natl. Acad Sci. USA, 2004 101, 10446-10451.
19. Fahey,J.W. and Talalay,P. (1999) Antioxidant functions of sulforaphane: a potent inducer of Phase II detoxication enzymes. Food Chem. Toxicol., 37, 973-979.
20. Dinkova-Kostova,A.T., Liby, K.T., Stephenson, K.K., Holtzclaw,W.D., Gao,X., Suh,N., Williams,C., Risingsong,R., Honda,T., Gribble,G.W., Spom,M.B, and Talalay,P. (2005) Extremely potent triterpenoid inducers of the phase 2 response: correlations of protection against oxidant and inflammatory stress. Proc. Natl. Acad. Sci. USA, 102, 4584-4589.
21. Zhang,Y., Kensler,T.W., Cho,C.G., Posner,G.H. and Talalay,P. (1994) Anticarcinogenic activities of sulforaphane and structurally related synthetic norbornyl isothiocyanates. Proc. Natl. Acad. Sci. USA, 91, 3147-3150.
22. Chung,F.L., Conaway,C.C., Rao,C.V. and Reddy,B.S. (2000) Chemoprevention of colonic aberrant crypt foci in Fischer rats by sulforaphane and phenethyl isothiocyanate. Carcinogenesis, 21, 2287-2291.
23. Fahey,J.W., Haristoy,X., Dolan,P.M., Kensler,T.W., Scholtus,I, Stephenson,K.K., Talalay,P. and Lozniewski,A. (2002) Sulforaphane inhibits extracellular, intracellular, and antibiotic-resistant strains of Helicobacter pylori and prevents benzo[a]pyrene-induced stomach tumors. Proc. Natl. Acad. Sci. USA, 99, 7610-7615.
24. Lu,Y.P., Lou,Y.R., Lin,Y., Shih,W.J., Huang,M.T., Yang,C.S. and Conney,A.H. (2001) Inhibitor effects of orally administered green tea, black tea, and caffeine on skin carcinogenesis in mice previously treated with ultraviolet B light (high-risk mice): relationship to decreased tissue fat. Cancer Res., 61, 5002-5009.
25. Lu,Y.P., Lou,Y.R., Xie,J.G., Peng,Q.Y., Liao,J., Yang,C.S., Huang,M.T. and Conney,A.H. (2002) Topical applications of caffeine or (-)-epigallocatechin gallate (EGCG) inhibit carcinogenesis and selectively increase apoptosis in UVB-induced skin tumors in mice. Proc. Natl. Acad. Sci. USA, 99, 12455-12460.
26. Troyer,J.K., Stephenson,K.K. and Fahey,J.W. (2001) Analysis of glucosinonlates from broccoli and other cruciferous vegetables by hydrophilic interaction liquid chromatography. J. Chromatogr. A, 919, 299-304.
27. Shapiro,T.A., Fahey,J.W., Wade,K.L., Stephenson,K.K. and Talalay,P. (2001) Disposition of chemoprotective glucosinolates and isothiocyanates of broccoli sprouts. Cancer Epidemiol. Biomark. Prevent., 10, 501-508:
28. Zhang,Y., Wade,K.L., Prestera,T. and Talalay,P. (1996) Quantitative determination of isothiocyanates, dithiocarbamates, carbon disulfide, and related thiocarbonyl compounds by cyclocondensation with 1,2-benzenedithiol. Anal. Biochem., 239, 160-167.
29. Prochaska,H.J. and Santamaria,A.B. (1988) Direct measurement of NAD(P)H:quinone reductase from cells cultured in microtiter wells: a screening assay for anticarcinogenic enzyme inducers. Anal. Biochem., 169, 328-336.
30. Fahey,J.W., Dinkova-Kostova.A.T., Stephenson,K.K. and Talalay,P. (2004) The "Prochaska" microtiter plate bioassay for inducers of NQO1. Methods Enzymol., 382, 243-258.
31. Anderson,M.E. (1985) Determination of glutathione and glutathione disulfide in biological samples. Methods Enzymol., 113, 548-555.
32. Richie,J.P. Jr., Skowronski,L., Abraham,P. and Leutzinger,Y. (1996) Blood glutathione concentrations in a large-scale human study. Clin. Chem., 42, 64-70.
33. Suh,N., Honda,T., Finlay,H.J., Barchowsky,A., Williams,C., Benoit,N.E., Xie,Q.W., Nathan,C., Gribble,G.W. and Sporn,M.B. (1998) Novel triterpenoids suppress inducible nitric oxide synthase (iNOS) and inducible cyclooxygenase (COX-2) in mouse macrophages. Cancer Res., 58, 717-723.
34. Ye,L. and Zhang,Y. (2001) Total intracellular accumulation levels of dietary isothiocyanates determine their activity in elevation of cellular glutathione and induction of Phase 2 detoxification enzymes. Carcinogenesis, 22, 1987-1992.
35. LeBel,C.P., Ischiropoulos,H. and Bondy, S.C. (1992) Evaluation of the probe 2',7'-dichlorofluorescin as an indicator of reactive oxygen species formation and oxidative stress. Chem. Res. Toxicol., 5, 227-231.
36. Wei,H., Zhang,X., Wang,Y. and Lebwohl, M. (2002) Inhibition of ultraviolet light-induced oxidative events in the skin and internal organs of hairless mice by isoflavone genistein. Cancer Lett., 185, 21-29.
37. Setlow,R.B. and Carrier,W.L. (1966) Pyrimidine dimers in ultraviolet-irradiated DNA's. J. Mol. Biol., 17, 237-254.
38. Sarasin,A. (1999) The molecular pathways of ultraviolet-induced carcinogenesis. Mutat. Res., 428, 5-10.
39. Sander,C.S., Chang,H., Hamm,F., Elsner,P. and Thiele,J.J. (2004) Role of oxidative stress and the antioxidant network in cutaneous carcinogenesis. Int. J. Dermatol., 43, 326-335.
40. Nishigori,C., Yarosh,D.B., Donawho,C. and Kripke,M.L. (1996) The immune system in ultraviolet carcinogenesis. J. Investig. Dermatol. Symp. Proc., 1, 143-146.
41. Ehrhart,J.C., Gosselet,F.P., Culerrier,R.M. and Sarasin,A. (2003) UVB-induced mutations in human key gatekeeper genes governing signalling pathways and consequences for skin tumourigenesis. Photochem. Photobiol. Sci., 2, 825-834.
42. Hussein,M.R. (2005) Ultraviolet radiation and skin cancer: molecular mechanisms. J. Cutan. Pathol., 32, 191-205.
43. Asher,G., Lotem,J., Cohen,B., Sachs,L. and Shaul,Y. (2001) Regulation of p53 stability and p53-dependent apoptosis by NADH quinone oxidoreductase 1. Proc. Natl. Acad. Sci. USA, 98, 1188-1193.
44. Ross,D. (2004) Quinone reductases multitasking in the metabolic world. Drug Metab. Rev., 36, 639-654.
45. Iskander,K., Gaikwad,A., Paquet,M., Long II,D.J., Brayton,C., Barrios,R. and Jaiswal,A,K. (2005) Lower induction of p53 and decreased apoptosis in NQO1-null mice lead to increased sensitivity to chemical-induced skin carcinogenesis. Cancer Res., 65, 2054-2058.
46. Merk,H., Jugert,F., Bonnekoh,B. and Mahrle,G. (1991) Induction and inhibition of NAD(P)H: quinone reductase in murine and human skin. Skin Pharmacol., 4, 183-190.
47. Clairmont,A., Sies,H., Ramachandran,S., Lear,J.T., Smith,A.G., Bowers,B., Jones,P.W., Fryer,A.A. and Strange,R.C. (1999) Association of NAD(P)H:quinone oxidoreductase (NQO1) null with numbers of basal cell carcinomas: use of a multivariate model to rank the relative importance of this polymorphism and those at other relevant loci. Carcinogenesis, 20, 1235-1240.
48. Afaq,F. and Mukhtar,H. (2001) Effects of solar radiation on cutaneous detoxification pathways. J. Photochem. Photobiol, 63, 61-69.
49. Lu,Y.P., Lou,Y.R.,Yen,P., Mitchell,D., Huang,M.T. and Conney,A.H. (1999) Time course for early adaptive responses to ultraviolet B light in the epidermis of SKH-1 I mice. Cancer Res., 59, 4591-4602.
50. Katiyar,S.K., Afaq,F., Perez,A. and Mukhtar,H. (2001) Green tea polyphenol (-)-epigallo-catechin-3-gallate treatment of human skin inhibits ultraviolet radiation-induced oxidative stress. Carcinogenesis, 22, 287-294.
51. Pinnell,S.R. (2003) Cutaneous photodamage, oxidative stress, and topical antioxidant protection. J. Am. Acad. Dermatol., 48, 1-19.
52. Record,LR. and Dreosti,LE. (1998) Protection by black tea and green tea against UVB and UVA + B induced skin cancer in hairless mice. Mutat. Res., 422, 191-199.
53. Katiyar,S.K., Matsui,M.S. and Mukhtar,H. (2000) Kinetics of UV light-induced cyclobutane pyrimidine dimers in human skin in vivo: an immunohistochemical analysis of both epidermis and dermis. Photochem. Photobiol., 72, 788-793.
54. Chen C, Yu R, Owuor ED, Kong AN. (2000) Activation of antioxidant-response element (ARE), mitogen-activated protein kinases (MAPKs) and caspases by major green tea polyphenol components during cell survival and death. Arch. Pharm. Res., 23, 605-612. 2.
55. Zoete,V., Rougée,M., Dinkova-Kostova,A.T., Talalay,P. and Bensasson,R.V. (2004) Redox ranking of inducers of a cancer-protective enzyme via the energy of their highest occupied molecular orbital. Free Radic. Biol. Med., 36, 1418-1423.
56. Juurlink,B.H. (2001) Therapeutic potential of dietary phase 2 enzyme inducers in ameliorating diseases that have an underlying inflammatory component. Can. J. Physiol. Pharmacol., 79, 266-282.
57. Wu,L., Noyan Ashraf,M.H., Facci,M., Wang,R., Paterson,P.G., Ferrie,A. and Juurlink,B.H. (2004) Dietary approach to attenuate oxidative stress, hypertension, and inflammation in the cardiovascular system. Proc. Natl. Acad. Sci. USA, 101, 7094-7099.
**58**. Li,J., Lee,J.M. and Johnson,J.A. (2002) Microarray analysis reveals an antioxidant responsive element-driven gene set involved in conferring protection from an oxidative stress-induced apoptosis in IMR-32 cells. J. Biol. Chem., 277, 388-394.

## Claims

1. Sulforaphane or a sulforaphane analog for use in suppressing UV light-induced skin carcinogenesis, wherein said sulforaphane or sulforaphane analog is administered topically at a dose of 0.3 µmol/100µl to 1 µmol/100µl.

2. Use of sulforaphane or a sulforaphane analog for the preparation of a medicament for suppressing UV light-induced skin carcinogenesis, wherein said sulforaphane or sulforaphane analog is administered topically at a dose of 0.3 µmol/100µl to 1 µmol/100µl.

3. The sulforaphane or the sulforaphane analog of claim 1 or the use of claim 2, wherein said sulforaphane or sulforaphane analog is administered transdermally.

4. The sulforaphane or the sulforaphane analog of claim 1 or 3 or the use of claim 2 or 3, wherein said sulforaphane or sulforaphane analog is derived from broccoli sprouts.

5. The sulforaphane analog of any one of claims 1, 3 or 4 or the use of any one of claims 2 to 4, wherein said sulforaphane analog is selected from the group consisting of 6-isothiocyanato-2-hexanone, exo-2-acetyl-6-isothiocyanatonorbornane, exo-2-isothio-cyanato-6-methylsulfonylnorbornane, 6-isothiocyanato-2-hexanol, 1-isothiocyanato-4-dimethylphosphonylbutane, exo-2-(1'-hydroxyethyl)-5-isothiocyanatonorbornane, exo-2-acetyl-5-isothiocyanatonorbornane, 1-isothiocyanato-5-methylsulfonylpentane, cis-3-(methylsulfonyl)cyclohexylmethylisothiocyanate and trans-3- (methylsulfonyl)cyclohexylmethylisothiocyanate.

## Patentansprüche

1. Sulforaphan oder ein Sulforaphan-Analogon zur Verwendung bei der Unterdrückung von durch UV-Licht induzierter Hautkarzinogenese, wobei das Sulforaphan oder Sulforaphan-Analogon topisch in einer Dosis von 0,3 µmol/100 µl bis 1 µmol/100 µl verabreicht wird.

2. Verwendung von Sulforaphan oder eines Sulforaphan-Analogons für die Herstellung eines Medikaments zur Unterdrückung von durch UV-Licht induzierter Hautkarzinogenese, wobei das Sulforaphan oder Sulforaphan-Analogon topisch in einer Dosis von 0,3 µmol/100 µl bis 1 µmol/100 µl verabreicht wird.

3. Sulforaphan oder Sulforaphan-Analogon nach Anspruch 1 oder die Verwendung nach Anspruch 2, wobei das Sulforaphan oder Sulforaphan-Analogon transdermal verabreicht wird.

4. Sulforaphan oder Sulforaphan-Analogon nach Anspruch 1 oder 3 oder die Verwendung nach Anspruch 2 oder 3, wobei das Sulforaphan oder Sulforaphan-Analogon aus Brokkolisprossen gewonnen wird.

5. Sulforaphan-Analogon nach einem der Ansprüche 1, 3 oder 4 oder Verwendung nach einem der Ansprüche 2 bis 4, wobei das Sulforaphan-Analogon ausgewählt ist aus der Gruppe bestehend aus 6-Isothiocyanato-2-hexanon, exo-2-Acetyl-6-isothiocyanatonorbornan, exo-2-Isothiocyanato-6-methylsulfonylnorbornan, 6-Isothiocyanato-2-hexanol, 1-Isothio-cyanato-4-dimethylphosphonylbutan, exo-2-(1'-Hydroxyethyl)-5-isothiocyanato-norbornan, exo-2-Acetyl-5-isothiocyanatonorbornan, 1-Isothiocyanato-5-methylsulfonyl-pentan, cis-3-(Methylsulfonyl)cyclohexylmethylisothiocyanat und trans-3-(Methyl-sulfonyl)cyclohexylmethylisothiocyanat.

## Revendications

1. Sulforaphane ou analogue de sulforaphane, pour une utilisation dans la suppression de la cancérogenèse de la peau induite par la lumière UV, ledit sulforaphane ou analogue de sulforaphane étant administré par voie topique à une dose de 0,3 pmole/100 µl à 1 µmole/100 µl.

2. Utilisation de sulforaphane ou d'un analogue de sulforaphane pour la préparation d'un médicament pour supprimer la cancérogenèse de la peau induite par la lumière UV, ledit sulforaphane ou analogue de sulforaphane étant administré par voie topique à une dose de 0,3 ymole/100 _{II}I à 1 ymole/100 _{Il}l.

3. Sulforaphane ou analogue de sulforaphane selon la revendication 1, ou utilisation selon la revendication 2, ledit sulforaphane ou analogue de sulforaphane étant administré par voie transdermique.

4. Sulforaphane ou analogue de sulforaphane selon la revendication 1 ou 3, ou utilisation selon la revendication 2 ou 3, ledit sulforaphane ou analogue de sulforaphane étant dérivé de pousses de brocolis.

5. Analogue de sulforaphane selon l'une quelconque des revendication 1, 3 ou 4, ou utilisation selon l'une quelconque des revendications 2 à 4, ledit analogue de sulforaphane étant sélectionné dans le groupe constitué de la 6-isothiocyanato-2-hexanone, de l'exo-2-acétyl-6-isothiocyanatonorbornane, de l'exo-2-isothiocyanato-6-méthylsulfonylnorbornane, du 6-isothiocyanato-2-hexanol, du 1-isothiocyanato-4-diméthylphosphonylbutane, de l'exo-2-(1'-hydroxyéthyl)-5-isothiocyanatonorbornane, de l'exo-2-acétyl-5-isothiocyanatonorbornane, du 1-isothiocyanato-5-méthylsulfonylpentane, du cis-3-(méthyl-sulfonyl)cyclohexylméthylisothiocyanate et du trans-3-(méthylsulfonyl)-cyclohexylméthylisothiocyanate.
